# EUROPÄISCHE PATENTSCHRIFT

(11) **EP 2 346 385 B1**
(45) Veröffentlichungstag und Bekanntmachung des Hinweises auf die Patenterteilung: **13.07.2016**
(21) Anmeldenummer: 09767927.8
(22) Anmeldetag: 01.10.2009
(51) Int. Cl.: A61B 1/04, A61B 1/07, A61B 1/00, G02B 6/36, G02B 7/24, A61B 1/055, A61B 1/06

(54) **ENDOSKOP**
ENDOSCOPE
ENDOSCOPE

(30) Priorität: 02.10.2008 DE 102008049922
(43) Veröffentlichungstag der Anmeldung: 27.07.2011
(73) Patentinhaber: Karl Storz GmbH & Co. KG, 78532 Tuttlingen (DE)
(72) Erfinder: IRION, Klaus, M., 78576 Liptingen (DE); EHRHARDT, André, 78573 Wurmlingen (DE); KRATTIGER, Beat, CH-8222 Beringen (CH)
(86) Internationale Anmeldenummer: PCT/DE2009/001368
(87) Internationale Veröffentlichungsnummer: WO 2010/037374

(56) Entgegenhaltungen:
- DE-A1-102006 053 487
- DE-C1- 3 914 825
- US-A- 4 905 082
- US-A- 6 063 024

## Beschreibung

Die vorliegende Erfindung betrifft ein Endoskop nach dem Oberbegriff von Anspruch 1 sowie eine Endoskopoptik, ein Kameramodul und eine Kupplung zur Verwendung in einem solchen Endoskop.

Endoskope werden heute für eine Vielzahl von Anwendungen in Medizin und Technik verwendet. Endoskope umfassen typischerweise einen langerstreckten Schaft, der zur Einführung in einen Hohlraum geeignet ist, und in dem ein Bildleitsystem enthalten ist zur Weiterleitung eines endoskopischen Bildes vom distalen (beobachterfernen) Ende des Endoskops zum proximalen (beobachternahen) Ende. Als Bildleitsysteme sind insbesondere Linsensysteme, die eine Mehrzahl von Stablinsen umfassen können, und geordnete Bündel von Lichtleitfasern bekannt. Da in der Regel in dem beobachteten Hohlraum nicht ausreichend Licht vorhanden ist, ist ferner ein Lichtleitsystem vorgesehen, um Beleuchtungslicht an das distale Ende des Endoskops zu transportieren, wo es zur Beleuchtung des Hohlraums genutzt wird. Zur Einleitung des Beleuchtungslichts in die Endoskopoptik ist oft in der Nähe des proximalen Endes ein Anschluss zum Anschließen eines Lichtleitkabels vorhanden, mit dem das Beleuchtungslicht von einer separaten Lichtquelle zugeführt wird.

Am proximalen Ende sind Endoskope oft mit einem Okular für einen visuellen Einblick versehen. Immer häufiger wird jedoch eine elektronische Bildaufnahme vorgezogen, für die am proximalen Ende ein elektronischer Bildaufnehmer zur Umsetzung des weitergeleiteten optischen Bildes in ein Videobild vorgesehen sein kann. Eine elektronische Bildauf-nahme hat eine Vielzahl von Vorteilen gegenüber einer rein visuellen Betrachtung. So ist beispielsweise die Bildwiedergabe auf beliebig angeordneten Videoschirmen möglich, die eine ermüdungsfreie Betrachtung und eine problemlose Teilnahme weiterer Beobachter gestatten. Darüber hinaus bestehen vielfältige Möglichkeiten zur Weiterleitung, Bearbeitung und Speicherung von Videobildern.

Häufig ist es wünschenswert, den elektronischen Bildaufnehmer von einer Endoskopoptik trennen und wieder mit dieser oder einer anderen Endoskopoptik verbinden zu können. Dies kann beispielsweise bei einer endoskopischen Operation notwendig sein, wenn unterschiedliche Blickrichtungen innerhalb des betrachteten Hohlraums realisiert werden sollen. In diesem Fall wird der Bildaufnehmer beibehalten, jedoch während der Operation die Endoskopoptik gewechselt. Auch aus Gründen der Handhabung beim Einführen des Endoskops in einen Hohlraum, wie auch der Instandhaltung und Rationalisierung oder auch beispielsweise zur Verwendung von Einweg-Endoskopoptiken kann es vorteilhaft sein, Endoskopoptik und Bildaufnehmer voneinander trennen und wieder miteinander verbinden zu können. Zu diesem Zweck ist es bekannt, den elektronischen Bildaufnehmer in einem Kameramodul anzuordnen, das ein Gehäuse, Anschlüsse für Versorgungs- und Datenleitungen und einen Anschluss zur lösbaren Verbindung mit einer Endoskopoptik aufweist.

Dabei ist es aus Gründen der Handhabung, insbesondere bei endoskopischen Operationen, besonders vorteilhaft, wenn für das Wechseln der Endoskopoptik nur ein einziger Vorgang zum Lösen bzw. Verbinden erforderlich ist. Zu diesem Zweck sind Universalkupplungen zur lösbaren Verbindung von Endoskopoptik und Kameramodul entwickelt worden, bei denen sowohl ein Anschluss des Bildleitsystems als auch des Lichtleitsystems erfolgt. Es ist dann möglich, mit einem einzigen Verbindungsvorgang eine Endoskopoptik zu wechseln. Hierbei wird auch das Beleuchtungslicht durch das Kameramodul geführt oder im Kameramodul erzeugt.

Eine Universalkupplung dieser Art ist in der DE 197 15 510 A1 beschrieben. Hierbei stehen von einem Kupplungsende einer Endoskopoptik zwei zylindrische Zapfen vor, von denen einer einen proximalen Endabschnitt des Bildleitsystems und einer einen proximalen Endabschnitt des Lichtleitsystems enthält. Im Kameramodul sind zwei entsprechende Ausnehmungen vorgesehen, in die die beiden Zapfen beim Verbinden der Endoskopoptik mit dem Kameramodul eindringen, wobei der Boden der einen Ausnehmung eine Verbindung zum Bildaufnehmer des Kameramoduls und der Boden der anderen Ausnehmung eine Verbindung zu einem Lichtleiter aufweist, in den Beleuchtungslicht eingespeist werden kann. Ferner sind mechanische Mittel zum Verriegeln der Kupplung vorgesehen.

In der Endoskopie, insbesondere in der endoskopischen Diagnostik und Chirurgie, tritt jedoch häufig der Fall ein, dass die Endoskopoptik um die Längsachse des Endoskopschafts gedreht wird, beispielsweise aus Gründen der Handhabung eines gemeinsam mit der Endoskopoptik benutzten chirurgischen Instruments, oder auch, um bei einer Schrägblickoptik, etwa einer 30°-Optik, einen anderen Bereich einer Wand des beobachteten Hohlraums zu sehen. In diesem Fall dreht sich bei einem starr mit der Endoskopoptik verbundenen Videobildaufnehmer das endoskopische Videobild entgegengesetzt zur Drehrichtung der Endoskopoptik. Dies stellt für einen Operateur eine erhebliche Erschwernis bei der Orientierung in dem beobachteten Hohlraum dar.

Es ist deshalb vorgeschlagen worden, die Bildrotation bei Rotation der Endoskopoptik durch eine Gegendrehung des Bildaufnehmers auszugleichen. So wird in der US 6,097,423 ein Videoendoskop beschrieben, bei dem mit Hilfe eines Beschleunigungsaufnehmers eine auf die Schwerkraft bezogene Referenzrichtung bestimmt und eine motorische Rotation des Bildaufnehmers durchgeführt wird, um die Bildrotation zu vermeiden. Das Kameramodul selbst ist durch eine Universalkupplung der oben beschriebenen Art starr mit der Endoskopoptik verbunden. Diese Lösung ist mit einem relativ hohen elektronischen und mechanischen Aufwand verbunden.

In der US-Patentschrift US 6,063,024 wird ein Beobachtungsapparat offenbart, der beispielsweise als Endoskop eingesetzt werden kann.

Einige Ausführungsformen umfassen ein Verbindungsmittel mit einem Schraubgewinde, wodurch der Schaft lösbar aber nicht drehbar verbunden ist. Da in US 6,063,024 J nur eine gerade Ausblickoptik beschrieben wird, gibt es auch keine Notwendigkeit dass der Schaft zum Rest des Beobachtungsapparates drehbar gelagert ist.

Gemäß DE 39 14 825 C1 kann das Kameramodul auch drehbar mit der Endoskopoptik verbunden sein. Während das aus Lichtleitfasern bestehende Bildleitsystem über einen axialen Kanal auf einen Bildwandler gerichtet ist, ist das Lichtleitsystem über einen zum axialen Kanal konzentrisch angeordneten Ring mit den von einer externen Lichtquelle versorgten Lichtleitern verbunden. Dies ermöglicht, durch eine manuelle Drehung eine Rotation der Endoskopoptik auszugleichen bzw. bei einer Rotation der Endoskopoptik das Kameramodul festzuhalten, um eine Rotation des Videobildes zu vermeiden. Auch diese Lösung ist mit einem hohen Aufwand verbunden, da zur Übertragung zur Bildinformation und des Beleuchtungslichts zwei unterschiedliche Kanäle jeweils exakt verbunden werden müssen. Außerdem kann der koaxiale Lichtübertragungsring bei kleinkalibrigen Endoskopen nur relativ dünn ausgeführt werden, und es kann zu hohen Koppelverlusten kommen.

In der US-Patentschrift US 4,905,082 wird ein starres Endoskop offenbart, bei dem innerhalb des Schafts die Bildübertragung durch ein Relaislinsensystem und die Übertragung des Beleuchtungslichts durch ein Lichtleitsystem aus Lichtleitfasern erfolgt, die das Relaislinsensystem umgeben. Ein proximaler Endabschnitt des Relaislinsensystems steht, zusammen mit einem lichtundurchlässigen Rohr, das dass Relaislinsensystem aufnimmt, axial über die ringförmige proximale Endfläche des Lichtleitsystems vor. Eine Abbildungseinheit, die einen CCD-Bildaufnehmer oder ein Okular umfasst, weist eine entsprechende axiale Aussparung für die Aufnahme des vorstehenden Abschnitts des Relaislinsensystems auf sowie einen ringförmigen Lichtleiter, der der Endfläche des Lichtleitsystems des Schafts gegenüber steht. Die Abbildungseinheit ist mit dem Schaft lösbar und drehbar verbindbar. Dadurch, dass der konzentrische Lichtübertragungsring das Bildübertragungssystem umgibt, kann dieser bei kleinkalibrigen Endoskopen nur relativ dünn ausgeführt werden. Zudem ist die optisch-mechanische Ausführung aufwendig, und es kann zu hohen Koppelverlusten und zu starken Intensitätsschwankungen bei der Rotation kommen.

In DE 10 2006 053 487 A1 ist ein endoskopisches System offenbart, in dem die Beleuchtung über einen distalseitig angeordneten Fluoreszenzumsetzer erfolgt. Eine Videokamera ist ebenfalls in den distalen Endbereich des Schafts integriert. Aus DE 102 46 521 A1, US 2001/0029316 A1 und US 2003/0064025 A1 ist es bekannt, Beleuchtungslicht und vom beobachteten Objektfeld emittiertes Licht in voneinander getrennten Spektralbereichen durch ein gemeinsames Licht- und Bildübertragungssystem im gesamten Endoskopschaft zu führen. Eine lösbare und drehbare Verbindung zwischen einer Kameraeinheit und dem Schaft ist nicht vorgesehen.

Es ist die Aufgabe der vorliegenden Erfindung, ein Endoskop anzugeben, bei dem das Herstellen einer lösbaren und drehbaren Verbindung zwischen Endoskopoptik und Kameramodul zur Übertragung des Beleuchtungslichts und des endoskopischen Bilds möglich ist, wobei die vorstehend genannten Nachteile vermieden werden.

Diese Aufgabe wird durch ein Endoskop mit den Merkmalen des Anspruchs 1 gelöst.

Eine zu einem erfindungsgemäßen Endoskop gehörende Endoskopoptik weist insbesondere einen in einen Hohlraum einführbaren Schaft auf. Die Endoskopoptik umfasst ein optisches Lichtübertragungssystem zur Übertragung von Beleuchtungslicht vom proximalen Ende zum distalen Ende, wo dieses aus der Endoskopoptik austreten und zur Beleuchtung des Hohlraums und eines in diesem befindlichen Objektfelds dienen kann. Ferner umfasst die Endoskopoptik ein optisches Bildübertragungssystem zur Übertragung eines Bildes des Objektfelds vom distalen zum proximalen Ende. Bildübertragungssysteme dieser Art umfassen üblicherweise Linsensysteme oder geordnete Bündel von Lichtleitfasern. Die dabei zur Bildweiterleitung verwendeten Linsen können insbesondere Stablinsen aus homogenen Materialien und mit gekrümmten End- oder Zwischenflächen sein oder Stablinsen aus optisch inhomogenem (Gradientindex-) Material mit zumeist ebenen Endflächen. Als Lichtleitfasern kommen sowohl Mono- wie Multimodefasern in Frage. Optische Licht- oder Bildübertragungssysteme sind häufig auch zur Weiterleitung von Licht in an das optische Spektrum anschließenden Bereichen im infraroten und im ultravioletten Teil des Spektrums geeignet, diese Bereiche werden hier daher ebenfalls als "optisch" verstanden.

Ein erfindungsgemäßes Endoskop umfasst weiterhin ein Kameramodul, das zumindest einen elektronischen Bildaufnehmer aufweist, der zur Aufnahme des Bilds, das über das Bildübertragungssystem und die Koppelstelle zum Kameramodul weitergeleitet wird, angeordnet ist. Der Bildaufnehmer bzw. Bildwandler kann beispielsweise ein CCD- , ein CMOS- bzw. Videochip sein, es können aber auch mehrere, ggf. unterschiedliche, Bildsensoren mit entsprechenden Lichtwegen vorgesehen sein. Ferner umfasst das Kameramodul eine Lichtzuführung zur Zuführung von Beleuchtungslicht in die Endoskopoptik, wobei dieses innerhalb des Kameramoduls erzeugt oder von außerhalb des Kameramoduls zur Lichtzuführung eingeleitet werden kann.

Endoskopoptik und Kameramodul können weitere mechanische, optische, elektrische und/oder elektronische Elemente aufweisen. So kann insbesondere im distalen Bereich der Endoskopoptik ein Endoskopobjektiv vorgesehen sein zur Erzeugung eines, vom Bildübertragungssystem zu übertragenden, Bild des Objektfelds auf einer Eingangsfläche des Bildübertragungssystems, beispielsweise der distalen Endfläche des geordneten Bündels von Lichtleitfasern. Im proximalen Bereich der Endoskopoptik kann eine Okularoptik angeordnet sein. Das Kameramodul kann eine Kameraoptik umfassen, ferner beispielsweise einen Lichtleiter- und einen Kameraanschluss mit entsprechenden Versorgungs- und Signalleitungen.

Endoskopoptik und Kameramodul können über eine Koppelstelle miteinander verbunden und wieder voneinander getrennt werden. Die Koppelstelle ist drehbar ausgebildet, so dass hierdurch eine Drehachse definiert wird, die vorzugsweise etwa parallel zu einer Längsachse des Schafts gerichtet ist, insbesondere koaxial zu dieser Längsachse verläuft. Im Bereich der Koppelstelle ist ein in Bezug auf die Drehachse axialer Kanal zur Übertragung des Bilds des Objektfelds von der Endoskopoptik zum Kameramodul und zur Übertragung des Beleuchtungslichts vom Kameramodul zur Endoskopoptik vorgesehen. Dieser ist bevorzugt zentral ausgebildet, d. h., die Drehachse verläuft selbst innerhalb des Kanals. Dabei kann der axiale Kanal durch optische und/oder mechanische Bauelemente realisiert sein, kann aber auch nur den Strahl- bzw. den Lichtweg zwischen Endoskopoptik und Kameramodul selbst bezeichnen.

Insbesondere kann ein proximaler Endabschnitt des Bildübertragungssystems der Endoskopoptik in dem axialen Kanal aufgenommen sein oder bildet selbst diesen Kanal, ebenso ein proximaler Endabschnitt des Lichtübertragungssystems. Auf der Seite des Kameramoduls ist der axiale Kanal optisch verbunden mit Bildaufnehmer und Lichtzuführung, so dass das durch den axialen Kanal übertragene Bild des Objektfelds von Bildaufnehmer aufgenommen und das von der Lichtzuführung zugeführte Beleuchtungslicht in den axialen Kanal eingeleitet werden kann. Zur Übertragung des Beleuchtungslichts und des endoskopischen Bilds durch den axialen Kanal kann dieser insbesondere eine vorbestimmte Luftstrecke aufweisen, an der Endoskopoptik und Kameramodul miteinander verbunden und gegeneinander gedreht werden können. Es kann aber auch vorgesehen sein, dass auf Seiten der Endoskopoptik und/oder des Kameramoduls die jeweiligen Lichtwege in planen Endflächen enden, die bei einer Verbindung von Endoskopoptik und Kameramodul drehbar aneinander anliegen, oder es kann ein Übertragungsmedium, beispielsweise in flüssiger oder gelartiger Form vorgesehen sein, das einen Brechungsindex nahe dem der benachbarten optischen Elemente aufweist, um Reflexionen möglichst zu verhindern.

Dadurch, dass ein gemeinsamer Lichtweg für Beleuchtungslicht und Bild entlang eines axialen Kanals vorgesehen ist, kann mit geringem Aufwand eine freie Verdrehbarkeit der Endoskopoptik gegenüber dem im Kameramodul enthaltenen Bildaufnehmer erreicht werden. Hierfür ist eine axiale Anordnung des Kanals besonders vorteilhaft, es sind aber auch geringfügige Abweichungen von der axialen bzw. zur Drehachse koaxialen Anordnung möglich, auch kann beispielsweise eine zentrale Aussparung, z. B. für einen Zentrierstift oder ein Lager für die Drehung, vorgesehen sein.

Die Koppelstelle kann ein- oder mehrteilig ausgebildet sein. Insbesondere kann es auch möglich sein, dass die Verbindung zwischen Endoskopoptik und Kameramodul an einer Stelle trennbar ist, die keine Drehbarkeit erlaubt, und stattdessen die Drehbarkeit an einer anderen Stelle des Endoskops realisiert ist, die nicht trennbar sein muss. In diesem Fall ist der axiale Kanal insbesondere im Drehgelenk vorgesehen, während an der Trennungsstelle eine anders gestaltete Verbindung möglich sein kann.

Erfindungsgemäß ist das Endoskop derart ausgebildet, dass das Beleuchtungslicht in einem zentralen Bereich des axialen Kanals vom Kameramodul zur Endoskopoptik übertragen wird. Dabei ist der zentrale Bereich als zentraler Kanal innerhalb des axialen Kanals ausgebildet. Der zentrale Kanal enthält die Drehachse und ist in bevorzugter Weise zumindest abschnittsweise zylindrisch ausgebildet und koaxial zur Drehachse angeordnet. Hierdurch ist eine einfache, weitgehend verlustfreie und bei Rotation weitgehend schwankungsfreie Übertragung des Beleuchtungslichts möglich, das in der Regel eine hohe Leuchtdichte aufweist. Ferner ermöglicht die Übertragung des Beleuchtungslichts in einem zentralen Bereich auf einfache Weise die Übertragung des Beleuchtungslichts, wenn dieses nur über eine einzige oder eine geringe Anzahl von Lichtleitfasern übertragen wird, wie dies beispielsweise bei einer Beleuchtung durch Laserlicht vorteilhaft ist.

Erfindungsgemäß wird das Bild des Objektfelds in einem Bereich des axialen Kanals übertragen, der den zentralen Bereich radial umgibt, wobei der zur Bildübertragung genutzte Bereich des axialen Kanals ebenfalls zumindest abschnittsweise zylindrisch ausgebildet und koaxial zur Drehachse angeordnet sein kann. Hierdurch wird zumindest im Bereich der Koppelstelle eine räumliche Trennung der Übertragung des Beleuchtungslichts und des aufgenommenen endoskopischen Bilds erzielt. Insbesondere kann auf diese Weise eine räumliche Trennung des Beleuchtungslichts und des aufgenommenen Bilds über den gesamten oder nahezu den gesamten Übertragungsweg durch das Kameramodul und die Endoskopoptik erreicht werden. Hierdurch ist eine besonders verlustfreie Übertragung des Beleuchtungslichts bei einer ungestörten Übertragung des aufgenommenen endoskopischen Bilds möglich. Die räumliche bzw. spaziale Trennung von Beleuchtungslicht- und Bildübertragung ermöglicht es, außer UV auch Weißlicht, beispielsweise eines Superkontinuum-Lasers, oder Licht mehrerer Wellenlängen von einem oder mehreren Lasern, oder auch Licht von LED-, Halogen-, Xenon- Metall-Halid-Lichtquellen durch die Schnittstelle zu übertragen und zur Beleuchtung zu nutzen. Insbesondere kann ein gegenüber der Übertragung des Beleuchtungslichts größerer Anteil der Querschnittsfläche des axialen Kanals für die Bildübertragung genutzt werden. Dies hat den Vorteil einer größeren Schärfe des vom Bildaufnehmer aufgenommenen Bilds.

Erfindungsgemäß ist der zentrale Kanal lichtdicht koppelbar ausgebildet. Insbesondere kann der zentrale Kanal opake Hülsen, Fassungen und Führungen aufweisen, die bei einer Koppelung von Endoskopoptik und Kameramodul möglichst lichtdicht ineinander eingreifen und/oder aufeinander aufliegen, ggf. unter Verwendung flexibler Dichtelemente. Hierdurch kann eine weitestgehend lichtdichte Umhüllung des Übertragungswegs des Beleuchtungslichts erreicht werden, so dass das im den zentralen Kanal umgebenden Bereich des axialen Kanals übertragene Bild möglichst wenig durch Streulicht gestört wird.

Erfindungsgemäß ist der zentrale Kanal derart ausgebildet, dass das Beleuchtungslicht innerhalb des zentralen Kanals zumindest abschnittsweise über eine oder mehrere Lichtleitfasern übertragen werden. Die eine oder mehreren Lichtleitfasern im proximalen Endbereich der Endoskopoptik und im distalen Endbereich des Kameramoduls enden jeweils in Faserfassungen , die erfindungsgemäß federnd gegeneinander vorgespannt sind, so dass die Endflächen der Fasern bei einer Koppelung von Endoskopoptik und Kameramodul gegeneinander gedrückt werden. Hierdurch wird eine besonders einfache und verlustfreie Lichtübertragung ermöglicht. Die Verwendung nur einer oder weniger Lichtleitfasern, insbesondere bei Verwendung von Laserlicht, ermöglicht eine vorteilhafte Miniaturisierung des optischen Kontakts. Zum Ausgleich möglicher Toleranzen, beispielsweise wenn die Drehachse nicht exakt koaxial mit den Faserfassungen verläuft, kann mindestens eine der Faserfassungen beweglich gelagert sein.

Weiterhin ist es bevorzugt, dass die einander gegenüberstehenden Endflächen der Lichtleitfasern einen leichten Konkavschliff oder Schrägschliff aufweisen. Hierdurch kann bei der Rotation vermieden werden, dass Glasflächen aufeinander reiben und dadurch verkratzt werden könnten, was erhöhte Verluste bzw. erhöhtes Streulicht verursachen würde.

In einer bevorzugten Ausführungsform der Erfindung ist die Koppelstelle im Bereich eines parallelen Strahlengangs des Bildübertragungssystems und/oder einer Eintrittspupille des Kameramoduls angeordnet. Das Bildübertragungssystem der Endoskopoptik und ein Kameraobjektiv bzw. eine Kameralinse des Kameramoduls können dabei derart aufeinander abgestimmt sein, dass die Bildübertragung innerhalb der Koppelstelle, d. h., von der Endoskopoptik zum Kameramodul, über einen parallelen Strahlengang erfolgt. Die Koppelstelle bzw. das proximale Ende der Endoskopoptik bzw. das distale Ende des Kameramoduls kann insbesondere eine Eintrittspupille des Kameramoduls darstellen. Zumindest kann die Koppelstelle sich nahe einer Eintrittspupille befinden oder jedenfalls ausreichend weit entfernt von einer Bild- oder Zwischenbildebene.

Diese Maßnahme hat den Vorteil, dass eine eventuelle Abschattung des übertragenen endoskopischen Bilds, die durch Bauelemente zur Übertragung des Beleuchtungslichts durch den zentralen Bereich des axialen Kanals entstehen könnte, gering ist und sich nicht wesentlich auf die Qualität des vom Bildaufnehmer aufgenommenen Bilds auswirkt. Im Idealfall erfolgt eine Abschattung lediglich im Bereich eines parallelen Strahlengangs bzw. einer Eintrittspupille, so dass keine Verfälschung des Bilds auftritt, sondern lediglich eine geringfügige Abdunkelung entsprechend der abschattenden Fläche.

Gemäß einer bevorzugten Ausführungsform weist die Endoskopoptik eine Aufweitungsoptik auf zur Aufweitung eines Strahlengangs des Bildübertragungssystems zumindest im Bereich der Koppelstelle. Der Strahlengang im Kameramodul bzw. eine Kameralinse ist in diesem Fall entsprechend an den aufgeweiteten Strahlengang im Bereich der Koppelstelle angepasst. Hierdurch kann die Abdunkelung des vom Bildaufnehmer aufgenommenen Bilds verringert werden, da das Verhältnis der durch Einbauten zur Übertragung des Beleuchtungslichts abgeschatteten Fläche zur gesamten zur Bildübertragung genutzten Querschnittsfläche des axialen Kanals hierdurch verringert werden kann.

Gemäß einer weiteren bevorzugten Ausführungsform sind Endoskopoptik und Kameramodul derart ausgebildet, dass das Beleuchtungslicht in einem ersten Spektralbereich und das Bild des Objektfelds in einem hiervon getrennten zweiten Spektralbereich durch den axialen Kanal übertragbar sind. Der erste und der zweite Spektralbereich sind dabei derart gestaltet, dass sich beide nicht oder nur in einem unwesentlichen, für die Qualität des erhaltenen endoskopischen Bilds nicht störenden, Maße überlappen. Beide Spektralbereiche erstrecken sich dabei bevorzugt im sichtbaren Bereich des Spektrums oder schöpfen dieses gemeinsam aus, können aber auch dem infraroten oder ultravioletten Bereich angehören oder sich in diese Bereiche erstrecken.

Dadurch, dass das Beleuchtungslicht und das endoskopische Bild in voneinander getrennten Spektralbereichen übertragen werden, ist eine bidirektionale Übertragung entlang eines zumindest teilweise gemeinsamen Lichtwegs möglich, ohne dass die Qualität des endoskopischen Bilds durch Streuung oder Reflexe des zumeist wesentlich helleren Beleuchtungslichts beeinträchtigt wird. Gemäß einer besonderen Ausführungsform der Erfindung kann durch eine Kombination der oben beschriebenen räumlichen mit der hier beschriebenen spektralen Trennung der Übertragung von Beleuchtungslicht und Bild eine Störung des vom Bildaufnehmer empfangenen endoskopischen Bilds durch das Beleuchtungslicht praktisch vollständig vermieden werden.

Ein erfindungsgemäßes Endoskop kann beispielsweise auch für die photodynamische Diagnose (PDD) eingesetzt werden. Hierbei wird, in an sich bekannter Weise, Anregungslicht im kurzwelligen Bereich des optischen Spektrums erzeugt und über ein Lichtübertragungssystem auf biologisches Gewebe gerichtet. Aufgrund von gewebeeigenen oder zugeführten Fluoreszenzstoffen wird vom Gewebe eine langwelligere Fluoreszenzstrahlung erzeugt, die von der Art des Gewebes abhängig ist und insbesondere Gewebeentartungen erkennbar machen kann. Die Fluoreszenzstrahlung wird mit dem Endoskop aufgenommen, über ein Bildübertragungssystem einem Bildaufnehmer zugeführt und als elektronisches Bild, ggf. gemeinsam mit Weißlicht- oder weiteren Bildern, zur Diagnose dargestellt.

Ein erfindungsgemäßes Endoskop hat den Vorteil, dass eine Verwendung im Rahmen einer photodynamischen Diagnose möglich ist, wobei mit geringem Aufwand eine freie Drehbarkeit von Kameramodul und Endoskopoptik realisiert werden kann. Hierbei erfolgt die Umsetzung des in einem ersten, kurzwelligen Spektralbereich eingestrahlten Beleuchtungs- bzw. Anregungslichts in das Licht eines zweiten, langwelligeren Spektralbereichs, das mit dem Endoskop aufgenommen, weitergeleitet und elektronisch gewandelt wird, durch Fluoreszenz des Objekts, nämlich des Gewebes.

In einer bevorzugten Ausführungsform ist ein der Endoskopoptik zugeordneter erster Strahlteiler vorgesehen zur Auskopplung des Beleuchtungslichts aus dem Bildübertragungssystem. Der Strahlteiler ist insbesondere zur spektralen Trennung des ersten und des zweiten Spektralbereichs ausgebildet, beispielsweise als dichroitischer Spiegel oder als dichroitisches Prisma. Dadurch kann das Beleuchtungslicht aus dem Bildübertragungssystem ausgekoppelt werden und im weiteren Verlauf über ein hiervon separates Lichtübertragungssystem, insbesondere Lichtleitfasern, zum distalen Ende der Endoskopoptik geführt werden. Dies hat den Vorteil, dass im vom Strahlteiler gesehen distalen Verlauf des Bildübertragungssystems keine, das Bild eventuell störende Restfluoreszenz auftreten kann.

In besonders bevorzugter Weise ist im Strahlengang des Bildübertragungssystems eine Teilerfläche vorgesehen, die mit einer an sich bekannten spektral teilenden Beschichtung belegt ist. Diese kann beispielsweise innerhalb eines Teilerwürfels angeordnet sein, der aus zwei Rechtwinkelprismen zusammengesetzt ist, wobei die Hypotenusenfläche die Teilerschicht aufweist, während Bild und Beleuchtungslicht durch die Kathetenflächen übertragen werden. Hierdurch kann auf einfache Weise eine nahezu vollständige Trennung des ersten und des zweiten Spektralbereichs erzielt werden, so dass das über den axialen Kanal übertragene Beleuchtungslicht nahezu vollständig von dem hierzu distalen Abschnitt des Bildübertragungssystems entkoppelt werden kann. Zusätzlich können zur weiteren Verbesserung der Trennung spektrale Filter vorgesehen sein, die im Bild- und/oder Lichtübertragungssystem oder auch im Kameramodul vor dem Bildaufnehmer angeordnet sein können.

In einer besonders bevorzugten Ausführungsform ist weiterhin ein Umsetzer vorgesehen zur zumindest teilweisen Umsetzung des Beleuchtungslichts aus dem ersten in den zweiten Spektralbereich. Der Umsetzer ist innerhalb des Lichtübertragungssystems angeordnet, und zwar in Lichtrichtung nach, d. h., distal von dem ersten Strahlteiler. Dies hat den besonderen Vorteil, dass das Objektfeld mit einer Strahlung beleuchtbar ist, die ohne Störung durch das im ersten Spektralbereich übertragene Beleuchtungslicht vom Bildübertragungssystem aufgenommen und übertragen und vom Bildaufnehmer in ein darstellbares Bild umgesetzt werden kann. Der Umsetzer kann insbesondere derart gestaltet werden, dass eine ausreichende Helligkeit erzielbar ist, unabhängig von einer etwaigen Fluoreszenz im Objektfeld.

Insbesondere kann der Umsetzer in an sich bekannter Weise als Fluoreszenz-Umsetzer ausgebildet sein, wie beispielsweise in DE 10 2006 053 487 A1 beschrieben. Auf diese Weise kann eine Beleuchtungsstrahlung, die in einem ersten, kurzwelligen Spektralbereich übertragen wird, in eine Beleuchtungsstrahlung in einem zweiten, langwelligeren Spektralbereich umgesetzt werden. Hierfür kann ein Fluoreszenzkörper vorgesehen sein, oder es können geeignete Fluoreszenzstoffe auf die Lichtleitfasern aufgebracht bzw. in diese eingebracht werden. Durch geeignete Zusammensetzung der Fluoreszenzmaterialien kann eine spektrale Energieverteilung des Beleuchtungslichts erreicht werden, die nahezu weißem Licht entspricht.

Weiterhin ist es bevorzugt, das Beleuchtungslicht in das Lichtübertragungssystem mit kleiner numerischer Apertur (NA) einzukoppeln. Hierdurch kann verhindert werden, dass ein Anteil des Beleuchtungslichts im ersten Spektralbereich vom Fluoreszenzkörper in das Lichtübertragungssystem nach proximal zurückgestreut wird.

Andererseits kann es erwünscht sein, wenn ein gewisser Anteil des Beleuchtungslichts von dem Fluoreszenzkörper nicht von dem ersten in den zweiten Spektralbereich umgesetzt wird, sondern durch das weitere Lichtübertragungssystem auf das Objektfeld gelangt und nach Reflexion bzw. Streuung im Objektfeld in das Bildübertragungssystem aufgenommen wird. Ebenso könnte auch ein Anteil des Beleuchtungslichts im ersten Spektralbereich durch das Bildübertragungssystem auf das Objektfeld gelangen, wenn die spektrale Trennung durch die optischen Elemente im Bildübertragungssystem nicht vollständig ist. In diesem Fall kann ein Bildanteil im ersten Spektralbereich entstehen und vom Bildaufnehmer aufgenommen werden. Dies kann den Eindruck einer Weißlichtbeobachtung verbessern bzw. eine kontrastreichere Beobachtung des Objektfelds ermöglichen.

Der erste Strahlteiler kann gemäß einer Ausführungsform der Erfindung in einem distalen Endbereich der Endoskopoptik, insbesondere nahe dem distalen Ende des Schafts, vorgesehen sein. In diesem Fall erfolgt eine gemeinsame, entgegengerichtete Übertragung des Beleuchtungslichts und des aufgenommenen Bilds durch den Schaft, lediglich in einem distalen Endbereich sind für Beleuchtungslicht- und Bildübertragung verschiedene Lichtwege vorgesehen. Dies hat den Vorteil, dass für die Übertragung des Beleuchtungslichts von proximalen zum distalen Endbereich der Endoskopoptik keine zusätzlichen optischen Elemente benötigt werden, insbesondere kein Lichtleiter, der im Schaft untergebracht werden müsste und zum Außendurchmesser beitragen würde. Hierdurch ist es möglich, die Endoskopoptik über nahezu die gesamte Länge des Schafts mit einem geringeren Außendurchmesser auszubilden. Dies verringert insbesondere eine mögliche Traumatisierung eines Patienten bei einer endoskopischen Untersuchung.

Bevorzugt ist in dieser Ausführungsform der erste Strahlteiler zwischen Endoskopobjektiv und Bildweiterleiter angeordnet, insbesondere zwischen Endoskopobjektiv und distaler Stablinse bzw. distalem Ende des zur Bildweiterleitung dienenden Faserbündels. Dies hat den Vorteil, dass das helle Beleuchtungslicht nicht durch das Endoskopobjektiv geleitet wird, das in der Regel aus mehreren Linsen mit Grenz- und Kittflächen besteht. Das aufgenommene und weitergeleitete endoskopische Bild kann auf diese Weise nicht durch mögliche Fluoreszenz und/oder Reflexion bzw. Streuung im Endoskopobjektiv gestört werden. Der Umsetzer befindet sich nahe dem oder unmittelbar am distalen Ende des Schafts. In einer weiteren Fortbildung dieser Ausführungsform kann es auch vorgesehen sein, dass der distale Endbereich drehbar ausgebildet ist, wobei die Rotation vom proximalen Endbereich aus steuerbar sein kann. In diesem Fall kann die Trennstelle zum lösbaren Verbinden von Endoskopoptik und Kameramodul ohne Drehbarkeit ausgebildet sein.

In einer weiteren Ausführungsform der Erfindung ist der erste Strahlteiler in einem proximalen Endbereich der Endoskopoptik angeordnet. Dadurch sind die Lichtwege des Beleuchtungslichts und der Bildübertragung in der Endoskopoptik weitgehend getrennt und verlaufen nur im proximalen Endbereich gemeinsam. Dies hat den Vorteil, dass das übertragene Bild auch nicht durch mögliche Fluoreszenzeffekte oder Reflexion bzw. Streuung des hellen Beleuchtungslichts im Bildweiterleiter überlagert wird. Hierdurch kann daher ein ungestörtes, besonders kontrastreiches Bild des Objektfelds erhalten werden.

Weiterhin ist es vorteilhaft, dass das Kameramodul einen zweiten Strahlteiler umfasst zur Einkopplung des Beleuchtungslichts in den axialen Kanal, und damit in den gemeinsamen Lichtweg mit dem Bildübertragungssystem. Ebenso wird das durch den axialen Kanal übertragene Bild hierdurch von dem Beleuchtungslicht getrennt. Dies ist möglich aufgrund der spektralen Trennung des Beleuchtungslichts, das in einem ersten Spektralbereich übertragen wird, und des endoskopischen Bilds, das in einem zweiten Spektralbereich übertragen wird. Der zweite Strahlteiler kann zu diesem Zweck in ähnlicher Weise als dichroitischer Teiler ausgebildet sein wie der erste Strahlteiler und ist an die Apertur und geometrischen Gegebenheiten des Lichtwegs im Kameramodul angepasst.

In einer weiteren Ausführungsform ist die Lichtquelle in das Kameramodul integriert bzw. mit diesem zu einer Einheit mechanisch verbindbar. Hierdurch kann eine besonders kompakte Bauweise des Endoskops erzielt werden, die eine einfache Handhabung ermöglicht. Darüber hinaus ist zum Anschluss des Kameramoduls bzw. der Kameraeinheit und damit des Endoskops an eine Versorgungseinrichtung nur ein einziges elektrisches Kabel notwendig, das die Versorgungs-, Steuerungs- und Datenleitungen für die Lichtquelle und den oder die Bildaufnehmer enthalten kann. Dadurch werden auch der Anschluss und die Inbetriebnahme des Endoskops vereinfacht.

In bevorzugter Weise weist das Kameramodul eine Halbleiterlichtquelle zur Erzeugung des Beleuchtungslichts auf. Halbleiterlichtquellen sind kompakt und dadurch leicht in ein Kameramodul zu integrieren.

Die Halbleiterlichtquelle kann insbesondere einen Diodenlaser und/oder eine LED umfassen, sowie ggf. eine elektronische Schaltung zur Versorgung bzw. Ansteuerung. Insbesondere Diodenlaser haben den Vorteil einer schmalbandigen Lichterzeugung mit hoher Intensität, wobei das erzeugte Licht gut fokussierbar ist und mit kleiner numerischer Apertur auch in dünne Lichtleitfasern eingekoppelt werden kann. Eine hochgradig gebündelte Einkopplung mit geringer numerischer Apertur ist vorteilhaft für die Lichtübertragung und die Fluoreszenzumsetzung. Im Bereich der Strahlteiler kann auch ein konvergentes Strahlenbündel für die Übertragung vorteilhaft sein. Auch andere Halbleiterlichtquellen, wie lichtemittierende Dioden (LEDs) oder Superlumineszenzdioden sind kostengünstig verfügbar und ermöglichen eine ausreichende Lichterzeugung und Lichteinkopplung.

Halbleiterlichtquellen sind schnell schaltbar und weisen eine im Vergleich zu vielen anderen Lichtquellen relativ geringe Wärmeentwicklung auf. Um die entstehende Verlustwärme abzuführen, kann eine aktive Kühlung, beispielsweise durch ein Peltier-Element oder eine Flüssigkeitskühlung vorgesehen sein. Zur Verringerung der Kohärenz einer Laserlichtquelle können ggf. an sich bekannte Mittel, wie ein durch entsprechende elektrische Ansteuerung erzeugtes Rauschen und/oder mechanisch erzeugte Vibration, vorgesehen sein.

In einer weiteren Ausführungsform der Erfindung kann das Beleuchtungslicht stattdessen oder zusätzlich in einer externen Lichtquelle erzeugt und über ein Lichtleitkabel zum Kameramodul geleitet werden. Insbesondere kann am Kameramodul ein Anschluss für ein Lichtleitkabel vorgesehen sein, durch den das Beleuchtungslicht in das Kameramodul hinein und über die Lichtzuführung bzw. den ersten Strahlteiler in den axialen Kanal und hierdurch in das Lichtübertragungssystem der Endoskopoptik eingeleitet werden kann. Dies hat den Vorteil, dass die unvermeidliche Wärmeentwicklung bei der Lichterzeugung in einer vom Endoskop separaten Lichtquelle erfolgt und leichter, beispielsweise über einen Lüfter, abgeleitet werden kann. Dies ist auch deshalb vorteilhaft, weil die Lichtquelle außerhalb des sterilen Operationsbereichs angeordnet werden kann und nicht den hierfür notwendigen Einschränkungen unterliegt. Zudem können problemlos Lichterzeugungsmittel verwendet werden, die einen höheren Anteil an Verlustwärme erzeugen, wie etwa Xenonlampen, und/oder es kann eine insgesamt höhere Beleuchtungslichtstärke erzeugt werden. Außerdem kann ein ggf. notwendiger Wechsel des Lichterzeugungsmittels einfacher ausführbar sein. Natürlich können auch in einer externen Lichtquelle in vorteilhafter Weise Halbleiterlichtquellen und/oder Superkontinuum-Laserlichtquellen eingesetzt werden.

In einer bevorzugten Weiterbildung ist das Lichtleitkabel als kombiniertes elektrisch / optisches Verbindungskabel zur Verbindung mit einer externen Versorgungseinrichtung ausgebildet. Das Verbindungskabel umfasst mindestens ein elektrisches Kabel zur elektrischen Versorgung des Bildaufnehmers und ggf. zur Datenübertragung sowie ggf. zur Versorgung und Ansteuerung einer zusätzlichen, in das Kameramodul integrierten Lichtquelle. Weiterhin umfasst das Verbindungskabel einen Lichtleiter, insbesondere ein Faserbündel, zur Übertragung des in der externen Lichtquelle erzeugten Beleuchtungslichts zum Endoskop. Hierfür kann am Kameramodul ein kombinierter Anschluss vorgesehen sein, der optische und elektrische Verbindungselemente umfasst. Da somit für die Inbetriebnahme des Endoskops nur ein einziges Verbindungskabel anzuschließen ist, wird hierdurch die Handhabung weiter vereinfacht.

Die Versorgungseinrichtung umfasst insbesondere eine Kameraeinheit zur Versorgung des bzw. der Bildaufnehmer, zur Übernahme der aufgenommenen Bilddaten und ggf. zur Weiterverarbeitung, Weiterleitung und/oder Anzeige der Bilddaten. Weiterhin kann die Versorgungseinrichtung eine externe Lichtquelle umfassen zur Erzeugung des Beleuchtungslichts. Die Versorgungseinrichtung kann auch in kompakter Form ausgestaltet sein, ggf. als transportable Versorgungseinheit.

Alternativ oder zusätzlich zur Datenübertragung über ein Versorgungskabel kann auch eine drahtlose Datenübertragung, beispielsweise über ein Wireless Local Area Network (WLAN) vorgesehen sein. Das Kameramodul kann mit der hierfür notwendigen Prozessorkapazität ausgerüstet sein und auch eine unabhängige Stromversorgung, beispielsweise durch gängige Batterien oder neuartige Lithium-Ionen-Akkus, besitzen. Auch eine in das Kameramodul integrierte oder mit diesem mechanisch verbindbare Lichtquelle kann mit einer unabhängigen Stromversorgung ausgestattet sein. Hierdurch kann ein autarkes Endoskop mit einer weiter vereinfachten Handhabung geschaffen werden.

Das Kameramodul kann auch für eine Vorverarbeitung der empfangenen Bilddaten ausgebildet sein und/oder Bedienelemente zur Steuerung der Funktion des Bildaufnehmers und/oder der Lichtquelle aufweisen.

Zur Bedienung des Endoskops kann in vorteilhafter Weise an der Endoskopoptik ein Hebel vorgesehen sein, der die manuelle Drehung der Endoskopoptik zum Kameramodul ermöglicht. Insbesondere ist es vorteilhaft, wenn das Kameramodul derart ausgebildet ist, dass es mit einer Hand gehalten werden kann. Der Hebel kann dann so angeordnet sein, dass er mit einem Finger derselben Hand bedient werden kann, während das Kameramodul weiterhin sicher mit den übrigen Fingern der Hand gehalten werden kann.

In einer weiteren Ausführungsform ist alternativ hierzu oder zusätzlich ein Antrieb, insbesondere ein elektrischer Antrieb, vorgesehen, für eine aktive, ggf. automatische Rotation der Endoskopoptik relativ zum Kameramodul. Wenn das Kameramodul manuell oder auch in einer Einspannvorrichtung festgehalten wird, ist damit in besonders einfacher Weise eine Betrachtung verschiedener Bereiche eines Hohlraums, in den das Endoskop eingeführt ist, möglich.

In einer weiteren bevorzugten Ausführungsform der Erfindung umfasst der erste Spektralbereich Licht mit Wellenlängen unterhalb von 410 nm und der zweite Spektralbereich Licht mit Wellenlängen oberhalb von 410 nm. Insbesondere sind Strahlteiler bekannt, die eine praktisch vollständige Trennung bei dieser Wellenlänge ermöglichen. Darüber hinaus sind Diodenlaser mit einer Wellenlänge von 405 nm verfügbar, die Licht mit einer ausreichenden Energie zur Verfügung stellen. Das Beleuchtungslicht, das mit dieser Wellenlänge über den zweiten Strahlteiler in den axialen Kanal eingeleitet, durch den ersten Strahlteiler wieder vom Beobachtungsstrahlengang entkoppelt und im Lichtübertragungssystem zu einem Fluoreszenzumsetzer geführt wird, kann dort in an sich bekannter Weise in sichtbares Licht in einem breiten Spektralbereich mit Wellenlängen größer als 410 nm umgewandelt werden. Durch die spektrale Trennung bei etwa 410 nm geht für das aufgenommene Bild nur ein geringer Teil am kurzwelligen Ende des sichtbaren Spektrums verloren, d. h., es entfällt nur wenig Blauanteil im Bild. Auf diese Weise ist eine nahezu ungestörte Weißlichtbetrachtung des Objektfelds möglich. Alternativ kann auch beispielsweise eine Trennung bei 400 nm gewählt werden mit einer entsprechend kurzwelligeren Lichtquelle, um einen noch geringeren Blauanteil bei der Beobachtung zu verlieren.

Weiterhin ist es bevorzugt, dass mindestens ein Identifikations- bzw. Sicherheitselement vorgesehen ist zur Identifikation einer an das Kameramodul anzusetzenden Endoskopoptik bzw. zur Anpassung und/oder Freigabe der Lichterzeugung.

Endoskopoptiken unterschiedlicher Anwendungsgebiete, Längen, Durchmesser, Blickrichtungen und Gesichtsfeldwinkel können einen unterschiedlichen Energiebedarf zur Ausleuchtung des beobachteten Objektfelds haben. Um eine einfache und sichere Anpassung der in den Hohlraum eingekoppelten Beleuchtungsleistung zu erzielen, kann deshalb die Endoskopoptik mit einem Identifikationselement versehen sein, das beim Verbinden der Endoskopoptik mit dem Kameramodul automatisch erkannt wird und eine entsprechende Anpassung der Lichtleistung ermöglicht, beispielsweise durch Ansteuerung der Lichterzeugungsmittel oder durch Zuschaltung bzw. Abschaltung von LEDs. Das Identifikationselement kann hierzu mechanische, elektrische, elektronische oder andere Mittel aufweisen, die eine Identifikation der Endoskopoptik ermöglichen. Hierbei können beispielsweise bei Verwendung eines Transponders bzw. einer RFID von der Endoskopoptik zu einem im Kameramodul angeordneten Empfänger Daten übermittelt werden, die zur automatischen Anpassung der Strahlungsleistung verwendet werden können. Dies gestattet eine besonders einfache und vielseitige Verwendbarkeit des Kameramoduls.

Ferner kann automatisch registriert werden, ob die Verbindung mit einer Endoskopoptik besteht oder nicht. Solange keine Endoskopoptik mit dem Kameramodul verbunden ist, kann deshalb die Lichtleistung automatisch reduziert werden bzw. die Lichtquelle ausgeschaltet oder der Lichtweg blockiert werden. Erst nach Herstellung der Verbindung mit der Endoskopoptik wird bei dieser Ausführungsform die Strahlungsquelle freigegeben bzw. auf die geforderte Leistung gebracht. Ein solches Sicherheitselement bzw. ein derartiger Interlockmechanismus ist insbesondere vorteilhaft bei Verwendung einer Laserbeleuchtung, um eine Gefahr für Bedienpersonal und/oder Patienten zu vermeiden.

Es werden weiterhin eine Endoskopoptik, ein Kameramodul und eine Endoskopkupplung angegeben die zur Verwendung in einem erfindungsgemäßen Endoskop und zum Herstellen einer lösbaren und drehbaren Verbindung zwischen Endoskopoptik und Kameramodul geeignet sind.

Eine Endoskopoptik kann insbesondere auch die weiteren oben beschriebenen Merkmale aufweisen.

Insbesondere kann ein proximaler Endabschnitt des optischen Bildübertragungssystems und des Lichtübertragungssystems als axialer Kanal ausgebildet sein bzw. der axiale Kanal kann als Hülse ausgebildet sein, in der ein Endabschnitt des optischen Systems aufgenommen ist. Dies kann beispielsweise ein proximaler Endabschnitt einer Stablinse sein, die zumindest mit ihrer proximalen Endfläche im axialen Kanal angeordnet ist, oder auch eine proximale Endfläche eines Bündels von Lichtleitfasern oder ein Strahlteiler zur Entkopplung der Lichtwege von Bild- und Beleuchtungslichtübertragung. Auf diese Weise kann ein entsprechend ausgebildetes Kameramodul angeschlossen werden. Die Endoskopoptik kann auch als Einweg-Optik ausgebildet sein, die zur Verwendung mit einem entsprechenden Kameramodul, das zur mehrfachen Verwendung ausgelegt ist, verbindbar ist.

In einer bevorzugten Ausführungsform weist die Endoskopoptik einen zentralen Kanal innerhalb des axialen Kanals zur Übertragung des Beleuchtungslichts auf. In einer weiteren bevorzugten Ausführungsform ist das Lichtübertragungssystem der Endoskopoptik zur Übertragung von Beleuchtungslicht in einem ersten Spektralbereich und das Bildübertragungssystem der Endoskopoptik zur Übertragung des Bilds des Objektfelds in einem hiervon getrennten zweiten Spektralbereich ausgebildet.

Ein Kameramodul kann insbesondere auch die weiteren oben beschriebenen Merkmale aufweisen.

Insbesondere ist der elektronische Bildaufnehmer mit einem am distalen Ende des Kameramoduls angeordneten axialen Kanal derart verbunden, dass eine Übertragung eines von einer angeschlossenen Endoskopoptik zu übertragenden endoskopischen Bilds in einem zweiten Spektralbereich zum Bildaufnehmer erfolgen kann, so dass der Bildaufnehmer dieses in elektrische Signale bzw. in ein elektronisches Bild umwandeln kann. Hierfür können weitere optische Elemente zur Bildweiterleitung, Fokussierung, Filterung usw. vorgesehen sein. Gemäß einer Ausführungsform ist die Lichtzuführung derart mit dem axialen Kanal optisch verbunden, dass Beleuchtungslicht eines ersten Spektralbereichs in den axialen Kanal eingeleitet werden kann, zur Übertragung in eine anschließbare Endoskopoptik. Gemäß einer weiteren Ausführungsform ist innerhalb des axialen Kanals ein zentraler Kanal vorhanden, in den das Beleuchtungslicht eingeleitet werden kann, zur Übertragung in eine anschließbare Endoskopoptik. Auch hierfür können jeweils weitere optische Elemente vorgesehen sein, wie beispielsweise Spiegel, Strahlteiler oder Linsen. Diese optischen Elemente können in dem axialen bzw. zentralen Kanal angeordnet sein oder in diesen hineinreichen und/oder in einem axial angeordneten distalen Endabschnitt eine Endfläche zur Ein- bzw. Auskopplung von Licht aufweisen.

Eine Kupplung geeignet zur Verbindung von Endoskopoptik und Kameramodul eines erfindungsgemäßen Endoskops kann insbesondere auch die weiteren oben beschriebenen Merkmale aufweisen.

Eine erfindungsgemäße Kupplung kann als separates Bauelement ausgebildet sein, das beispielsweise eine lösbare Verbindung mit Endoskopoptik und/oder Kameramodul aufweist und eine Drehstelle besitzt, in deren Bereich der axiale Kanal angeordnet ist. Zur Verbindung von Endoskopoptik und Kameramodul kann es vorgesehen sein, dass Endflächen optischer Elemente, die den axialen Kanal bilden bzw. in diesem angeordnet sind, bündig aufeinander angesetzt werden, oder dass ein Zwischenraum zwischen diesen verbleibt, der beispielsweise mit Luft gefüllt sein kann. Es kann aber auch vorgesehen sein, dass ein solcher Zwischenraum mit einem beispielsweise flüssigen oder gelförmigen Medium gefüllt ist, das bevorzugt einen ähnlichen Brechungsindex aufweist wie die optischen Elemente. Die Endflächen können auch mit einer Anti-Reflex-Beschichtung versehen sein, wie auch andere optische Elemente in der Endoskopoptik und im Kameramodul.

Bei der Inbetriebnahme eines erfindungsgemäßen Endoskops können zunächst, sofern notwendig, die Versorgungseinrichtung eingeschaltet, die Kamera bzw. das Kameramodul mit der Versorgungseinrichtung verbunden und kalibriert, die Lichtquelle vorbereitet, Prozessoren bzw. Computerprogramme gestartet und Testläufe durchgeführt werden. Die Endoskopoptik wird sodann in einen Hohlraum, in dem die Untersuchung stattfinden soll, eingeführt, ggf. durch einen bereits vorher eingeführten Außenschaft. Das Kameramodul kann nun mit der Endoskopoptik in einem einzigen Vorgang verbunden werden, indem es aufgesetzt und die mechanische Verbindung hergestellt wird; hierdurch ist dann auch die optische Verbindung im Beleuchtungs- und im Beobachtungsstrahlengang hergestellt. Durch ggf. vorhandene Identifikations- und Sicherheitseinrichtungen kann beispielsweise die Beleuchtung automatisch für die Endoskopoptik angepasst werden, andernfalls kann die Beleuchtung von Bediener eingestellt und angeschaltet bzw. freigegeben werden.

Bei der Untersuchung des Hohlraums kann der Bediener die Endoskopoptik relativ zum Kameramodul drehen beispielsweise bei Vorhandensein eines entsprechenden Hebels mit Hilfe eines Fingers der Hand, die das Kameramodul und damit das Endoskop hält. Bei Verwendung einer Schrägblickoptik ist dabei ein Objektfeld seitlich zur Verlängerung der Schaftachse der Endoskopoptik sichtbar, das sich bei der Drehung um die Schaftachse verschiebt. Da das Kameramodul nicht mitrotiert, sondern festgehalten wird, bleibt das beispielsweise auf einem Videoschirm dargestellte Bild dabei aufrecht und rotiert nicht mit. Dies ermöglicht einem Bediener, beispielsweise einem Operateur, eine einfachere Orientierung in dem Hohlraum. So wird beispielsweise sowohl von der vom Bediener gesehen "linken" wie von der "rechten" Seite eines kugel- oder zylinderförmigen Hohlraums ein aufrecht stehendes Bild erhalten, bei dem "oben" und "unten" den tatsächlichen Richtungen im Hohlraum entsprechen, anders als bei einem mitrotierendem Bildaufnehmer. Übt der Bediener keine Rotationskraft auf die Endoskopoptik aus, so bleibt aufgrund der Reibung im Drehgelenk die relative Drehposition von Kameramodul und Endoskopoptik bestehen.

Soll während der Untersuchung eine andere Endoskopoptik eingesetzt werden, so wird das Kameramodul von der Optik getrennt, diese aus dem Hohlraum herausgezogen, eine neue Endoskopoptik, die beispielsweise eine andere Blickrichtung aufweist, eingeführt, und das Kameramodul mit dieser verbunden. Auch hierbei kann wieder eine manuelle oder automatische Anpassung der Beleuchtung an die Erfordernisse der Endoskopoptik erfolgen. Nach Beendigung der Untersuchung wird das Kameramodul von der Endoskopoptik getrennt, diese wird aus dem Hohlraum entnommen, das Kameramodul wird von der Versorgungseinrichtung getrennt, und Endoskopoptik und Kameramodul können einer getrennten Wiederaufbereitung für den nächsten Einsatz zugeführt werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, wobei die Erfindung durch die Ansprüche 1-10 definiert ist.

Weitere Aspekte der Erfindung ergeben sich aus der nachfolgenden Beschreibung eines bevorzugten Ausführungsbeispiels und der beigefügten Zeichnung.

Es zeigen, jeweils im Längsschnitt:
Fig. 1 ein Ausführungsbeispiel eines erfindungsgemäßen Endoskops;
Fig. 2 den proximalen Bereich eines weiteren Ausführungsbeispiels eines erfindungsgemäßen Endoskops;
Fig. 3 ein weiteres Ausführungsbeispiel eines erfindungsgemäßen Endoskops;
Fig. 4 eine als Strahlteiler ausgebildete Stablinse zur Verwendung in einem erfindungsgemäßen System;
Fig. 5a und 5b ein weiteres Ausführungsbeispiel eines erfindungsgemäßen Endoskops;
Fig. 6a und 6b in schematisierter Darstellung die Übertragung des Beleuchtungslichts und des Bilds im Bereich der Koppelstelle gemäß dem vorgenannten Ausführungsbeispiel;
Fig. 7a und 7b in schematisierter Darstellung die Übertragung des Beleuchtungslichts und des Bilds im Bereich der Koppelstelle gemäß einem weiteren Ausführungsbeispiel.

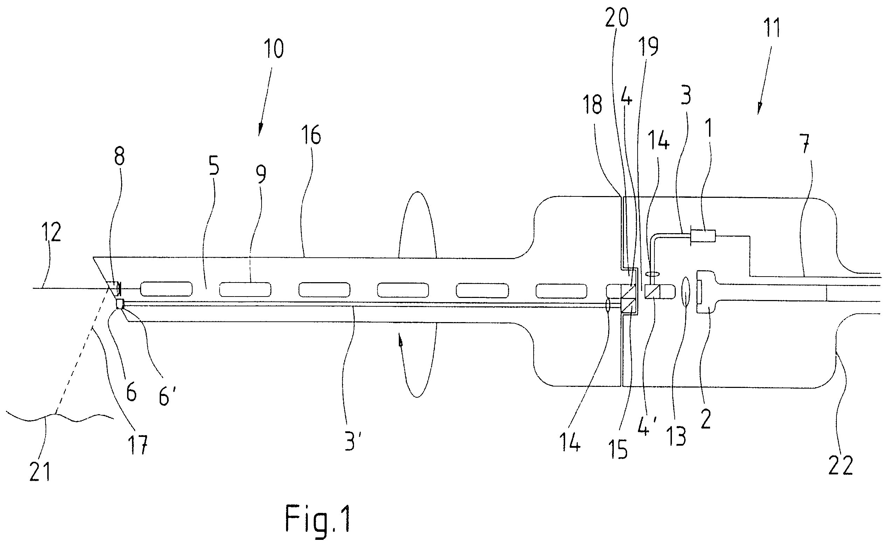

In Fig. 1 ist ein erfindungsgemäßes Endoskop dargestellt, das eine Endoskopoptik 10 und ein Kameramodul 11 umfasst. Endoskopoptik 10 und Kameramodul 11 sind an einer Koppelstelle 18 lösbar miteinander verbunden. Ein vom proximalen Ende der Endoskopoptik vorstehender Zapfen 20 ist zur Herstellung der optischen Verbindung und zur Zentrierung in einer entsprechenden Ausnehmung auf der distalen Seite des Kameramoduls aufgenommen. Weiterhin sind an der Koppelstelle 18 mechanische Mittel zur Herstellung einer lösbaren mechanischen Verbindung vorgesehen, beispielsweise kann am Kameramodul 11 eine Verriegelung vorgesehen sein, die an einer Hinterschneidung im Zapfen 20 eingreift und über ein Betätigungselement gelöst werden kann (nicht dargestellt). Die Koppelstelle 18 ist gleichzeitig zur freien Drehbarkeit von Kameramodul 11 und Endoskopoptik 10 ausgebildet, wobei ein gewisses Maß an Reibung erwünscht ist.

Das Kameramodul 11 umfasst einen Diodenlaser 1 zur Erzeugung einer Beleuchtungsstrahlung mit einer Wellenlänge von beispielsweise 405 nm mit einer optischen Ausgangsleistung von ca. 100 - 500 mW. Das von dem Diodenlaser 1 erzeugte Beleuchtungslicht wird in einen Multimode-Lichtleiter 3 eingekoppelt. Weiterhin umfasst das Kameramodul 11 einen elektronischen Bildaufnehmer 2, beispielsweise einen Videochip; stattdessen können auch mehrere Bildsensoren vorgesehen sein. Schließlich sind Versorgungs- und Datenleitungen 7 zur elektrischen Versorgung des Diodenlasers 1, des Bildaufnehmers 2 und zur Übertragung der aufgenommenen Bilddaten zu einer externen Kameraeinheit vorgesehen, sowie ein Gehäuse 22.

Die Endoskopoptik 10 umfasst ein optisches Bildübertragungssystem 5, das insbesondere eine Mehrzahl von Stablinsen 9 umfassen kann und in einem zylindrischen Schaft 16 aufgenommen ist. Der Schaft ist zur Einführung in einen Hohlraum, insbesondere in einen körperinneren Hohlraum, geeignet. Zur Verbindung mit einem zusätzlichen Außenschaft können im proximalen Endbereich des Schafts mechanische Verbindungselemente angeordnet sein (nicht dargestellt). Die Stablinsen können beispielsweise einen Durchmesser von 2,9 mm bei einem Außendurchmesser des Schafts von ca. 4 mm aufweisen. Ferner ist ein Endoskopobjektiv 8 vorgesehen, das von einem Objektfeld 21 ein Bild entwirft und in das Bildübertragungssystem 5 einleitet. Bei der dargestellten 30°-Optik schließt die Blickrichtung 17 zum aufgenommenen Objektfeld mit der Achse 12 des Schafts einen Winkel von 30° ein. Ferner umfasst die Endoskopoptik 10 einen weiteren Multimode-Lichtleiter 3', der ebenfalls im Schaft 16 aufgenommen ist.

Im Bereich der Koppelstelle bildet das Bildübertragungssystem 5 einen axialen Kanal 19, dessen Achse im Wesentlichen mit der Drehachse der Drehung der Kameraeinheit zur Endoskopoptik zusammenfällt, die hier mit der Achse 12 des Schafts zusammenfällt. In dem axialen Kanal stehen sich zwei plane Endflächen zweier Strahlteiler 4, 4' gegenüber, so dass eine ungestörte, weitgehend verlustfreie Lichtübertragung unabhängig von der Rotationsstellung stattfinden kann. Beide Strahlteiler sind hier als dichroitische Strahlteiler zur spektralen Trennung bei ca. 410 nm ausgebildet, insbesondere als Teilerwürfel, die aus jeweils 2 Rechteckprismen zusammengesetzt sind. Die Hypotenusenfläche ist mit der Teilerschicht 24 versehen, während die Kathetenflächen zur Lichtein- und -auskopplung benutzt werden.

Beide Strahlteiler sind in der in Fig. 1 dargestellten Ausführungsform derart angeordnet, dass nur Strahlung unterhalb einer Wellenlänge von 410 nm an der Teilerschicht reflektiert wird, Strahlung mit längeren Wellenlängen jedoch die Teilerschicht ohne Ablenkung passieren kann. Hierdurch wird ein Bild des Objektfelds 21, das vom Endoskopobjektiv 8 aufgenommen und vom Bildübertragungssystem 5 zum ersten Strahlteiler 4 weitergeleitet worden ist, im axialen Kanal in axialer Richtung zum Kameramodul 11 übertragen, passiert dort den zweiten Strahlteiler ebenfalls in axialer Richtung und wird über eine Kameralinse 13 auf den Bildaufnehmer 2 abgebildet. Voraussetzung hierfür ist, dass das Bild Licht mit Wellenlängen oberhalb von 410 nm umfasst.

Das von dem Diodenlaser erzeugte Licht einer Wellenlänge von 405 nm wird andererseits von der Teilerschicht des zweiten Strahlteilers 4' praktisch vollständig in axialer Richtung reflektiert und ebenso vollständig von der Teilerschicht des ersten Strahlteilers 4 aus der axialen Richtung auf einen Umlenkspiegel 15 geworfen, der das Beleuchtungslicht in den Lichtleiter 3' einkoppelt. Um anzudeuten, dass das vom Ende des Lichtleiters 3 ausgehende divergente Licht bevorzugt parallel durch den axialen Kanal übertragen und danach auf die Endfläche des Lichtleiters 3' fokussiert wird, sind in Fig. 1 symbolisch zwei Fokussierlinsen 14 eingezeichnet. Je nach Art der Einkopplung des Beleuchtungslichts in die Lichtleiter 3, 3' können die Fokussierlinsen auch entfallen, beispielsweise bei genügend geringer NA der erzeugten Laserstrahlung. Der Lichtleiter 3' kann beispielsweise einen Durchmesser von 0,1 mm haben.

Am distalen Ende des Lichtleiters 3' tritt das Beleuchtungslicht, das eine Wellenlänge von 405 nm aufweist, wieder aus dem Lichtleiter aus und trifft auf einen Fluoreszenzumsetzer 6. Dieser erzeugt Licht mit Wellenlängen, die größer als 405 nm sind, bevorzugt über einen breiten Wellenlängenbereich oberhalb von 410 nm, insbesondere nahezu weißes Licht. Je nach Abstrahlcharakteristik des Fluoreszenzumsetzers kann zur Strahlformung ein Reflektor 6' vorgesehen sein, der der erwünschten Beleuchtungsbreite und -richtung angepasst sein kann, um das zu beobachtende Objektfeld 21 möglichst vollständig und gleichmäßig auszuleuchten. Innerhalb des Objektfelds wird ein Anteil des Beleuchtungslichts durch Reflexion und Streuung zurückgeworfen und vom Endoskopobjektiv 8 aufgenommen. Auf diese Weise kann das Objektfeld im weißen Licht mit Wellenlängen oberhalb von 410 nm beobachtet und wie oben beschrieben ein entsprechendes Bild vom Bildaufnehmer aufgenommen und in elektrische Signale gewandelt werden.

Für den Fall, dass die spektrale Trennung in den beiden Strahlteilern 4, 4' nicht vollständig erfolgt, sondern ein Anteil des kurzwelligen Beleuchtungslichts im zweiten Strahlteiler 4' die Teilerschicht passiert, kann auf oder nahe der gegenüberliegenden Fläche des Strahlteilers 4' eine mattschwarze Beschichtung vorgesehen sein, um weitere Reflexionen im optischen System und damit eine Störung des aufgenommenen Bilds zu verhindern. Ebenso kann am ersten Strahlteiler 4 eine solche Schicht vorgesehen sein, um kurzwelliges Beleuchtungslicht, das durch den Strahlteiler axial hindurchgegangen ist und durch interne Reflexionen oder Streuung zum Strahlteiler 4 zurückgelangt, zu absorbieren, ebenso wie einen etwaigen Anteil des Bilds, der von der Teilerschicht im Strahlteiler 4 aus der Achse 12 heraus reflektiert wird. Um eine Störung des Bilds durch längerwelliges Licht, das durch Fluoreszenz innerhalb der optischen Elemente entstehen kann, zu vermeiden, ist es vorteilhaft, wenn zumindest diejenigen Elemente des Bildübertragungssystems, die auch vom Beleuchtungslicht durchlaufen werden, also insbesondere die Strahlteiler 4 und 4', aus reinem Quarzglas bestehen und dort keine organischen Kleber verwendet werden.

Das Gehäuse 22 des Kameramoduls 11 ist so ausgebildet, dass es vom Bediener mit einer Hand gehalten werden kann und dadurch das gesamte Endoskop sicher gehalten wird. Im proximalen Bereich der Endoskopoptik 10 kann beispielsweise ein seitlich vorstehender Hebel (nicht dargestellt) vorgesehen sein, der so ausgebildet und angeordnet ist, dass dieser vom Bediener mit einem Finger derselben Hand bewegt werden kann, um die Endoskopoptik gegenüber dem festgehaltenen Kameramodul zu verdrehen (in Fig. 1 durch einen gekrümmten Pfeil dargestellt). Je nach Blickrichtung und Blickfeld der Optik kann mit einer Schrägblickoptik hierbei schließlich ein Rundumblick und eine vollständige Untersuchung der Oberfläche des Hohlraums erzielt werden.

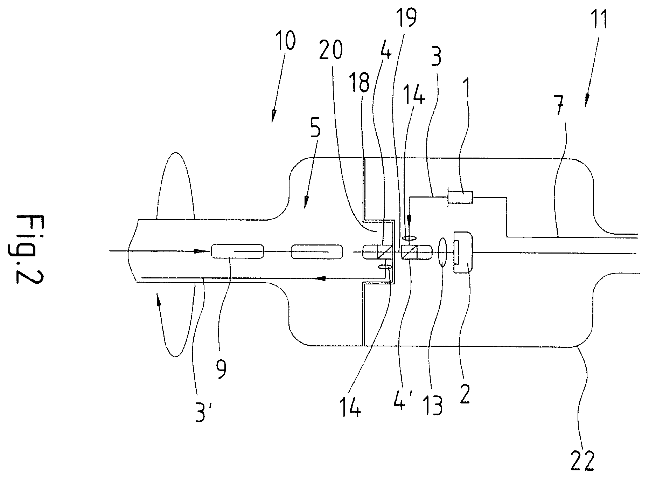

In Fig. 2 ist ein weiteres Ausführungsbeispiel der Erfindung dargestellt, wobei das Kameramodul 11 und der proximale Bereich der Endoskopoptik 10 gezeigt sind. Im Unterschied zu dem Ausführungsbeispiel der Fig. 1 ist hier kein Umlenkspiegel notwendig, da der Lichtleiter 3' zur Weiterleitung des Beleuchtungslichts innerhalb der Endoskopoptik derart angeordnet ist, dass das Beleuchtungslicht nach Reflexion im ersten Strahlteiler 4 ohne weitere Umlenkung in den Lichtleiter 3' eingekoppelt werden kann. Die Pfeile an den Lichtleitern 3 und 3' sowie im optischen Bildübertragungssystem 5 zeigen zur Verdeutlichung die jeweilige Richtung der Lichtübertragung.

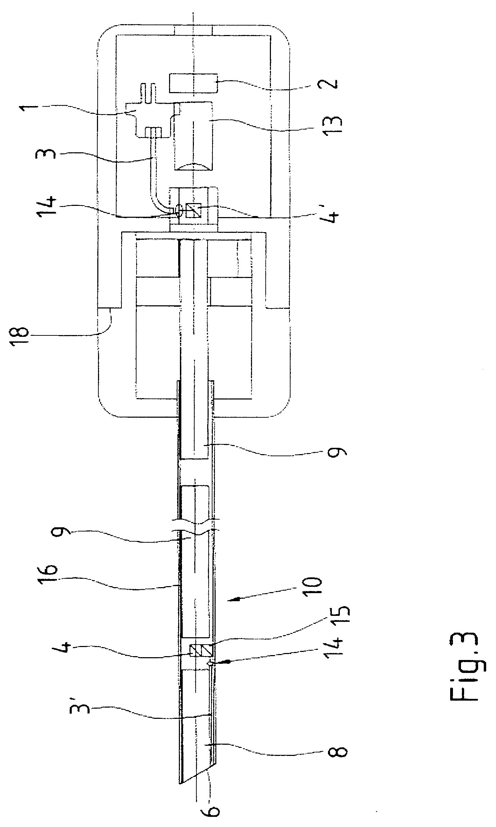

Beim erfindungsgemäße Ausführungsbeispiel der Fig. 3 ist der erste Strahlteiler 4 im distalen Endbereich des Schafts 16 zwischen dem Endoskopobjektiv 8 und der distalen Stablinse 9 der Endoskopoptik 10 angeordnet. Das Beleuchtungslicht wird nach Reflexion im ersten Strahlteiler 4 durch den Umlenkspiegel 15 und die Fokussierlinse 14 in den Lichtleiter 3' eingekoppelt.

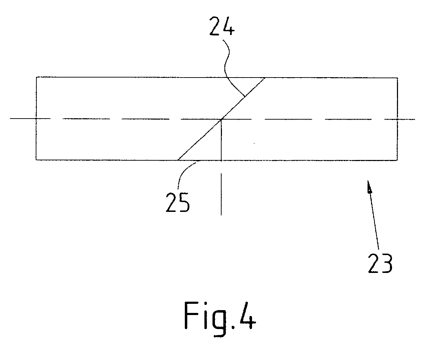

Insbesondere der erste Strahlteiler 4 kann auch als Stablinse ausgebildet sein, wie in Fig. 4 gezeigt ist. In diesem Fall ist innerhalb der Stablinse 23 eine Teilerschicht 24 vorgesehen, die nicht mittig angeordnet sein muss. Eine solche Stablinse lässt sich beispielsweise aus einer konventionellen Stablinse dadurch herstellen, dass diese insbesondere unter 45° durchgetrennt wird, auf einer der schrägen Flächen die Teilerschicht aufgebracht wird, und die beiden Teile der Stablinse wieder zusammengefügt werden. Hierbei sollten sowohl die Verwendung von organisch basiertem Kleber wie ein Brechungsindexsprung möglichst vermieden werden. Zur besseren Ein- und Auskopplung kann es weiterhin vorgesehen sein, dass zumindest im Bereich des seitlichen Strahlaustritts 25 eine ebene Fläche angeordnet ist, deren Größe mindestens dem Durchmesser des ausgekoppelten Strahls entspricht.

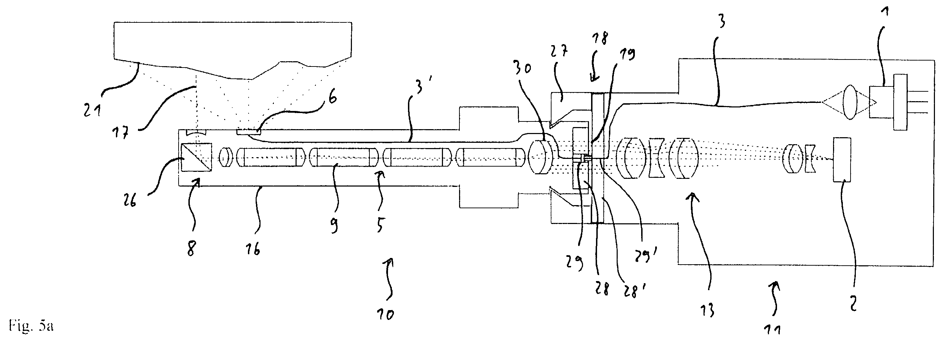

In Fig. 5a und 5b ist ein weiteres Ausführungsbeispiel eines erfindungsgemäßen Endoskops dargestellt, wobei in Fig. 5a die Endoskopoptik 10 und das Kameramodul 11 miteinander verbunden und in Fig. 5b voneinander getrennt gezeigt sind. Die Endoskopoptik 10 kann mit ihrem proximalen Ende mit dem distalen Endbereich des Kameramoduls 11 an der Koppelstelle 18 verbunden werden. Hierfür kann eine mechanische Haltevorrichtung 27 vorgesehen sein, die in Fig. 5a und 5b durch Klauen, die in entsprechende Aussparungen der Endoskopoptik 10 eingreifen, angedeutet ist. Das proximale Ende der Endoskopoptik 10 und das distale Ende des Kameramoduls sind jeweils durch Fenster 28, 28' abgeschlossen, die beispielsweise als planparallele Platten ausgebildet sein können. Durch die Fenster 28, 28' verläuft der axiale Kanal 19, durch den das Beleuchtungslicht und das Bild übertragen werden. Wie in Fig. 5a durch gestrichelte Linien angedeutet, ist der Strahlengang für die Bildübertragung im Bereich der Fenster 28, 28' parallel, und zueinander parallele, aber gegeneinander versetzte Strahlen werden im gleichen Punkt des Bilds, das auf dem Bildaufnehmer 2 entsteht, abgebildet. Die Drehachse entspricht der Achse des optischen Bildübertragungssystems 5 und ist in Fig. 5a und 5b nicht dargestellt. Der axiale Kanal 19 ist in diesem Fall durch den für die Übertragung des Bildes genutzten Bereich der Fenster 28, 28' bzw. die näherungsweise zylindrische Einhüllende des parallelen Strahlenbündels bestimmt.

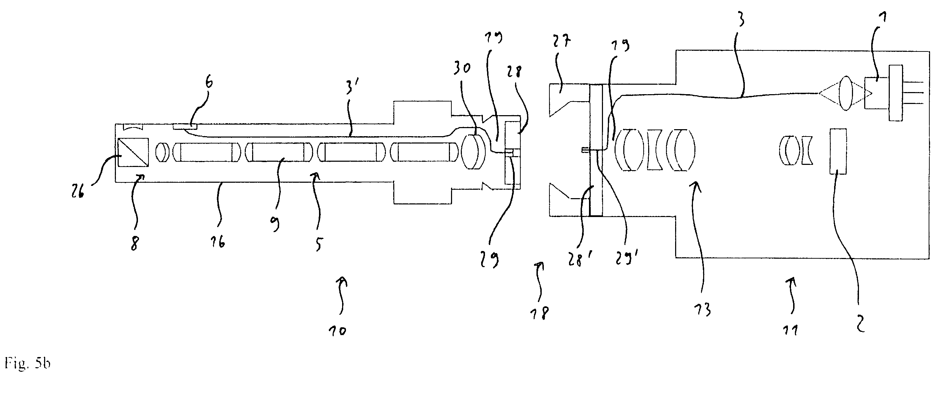

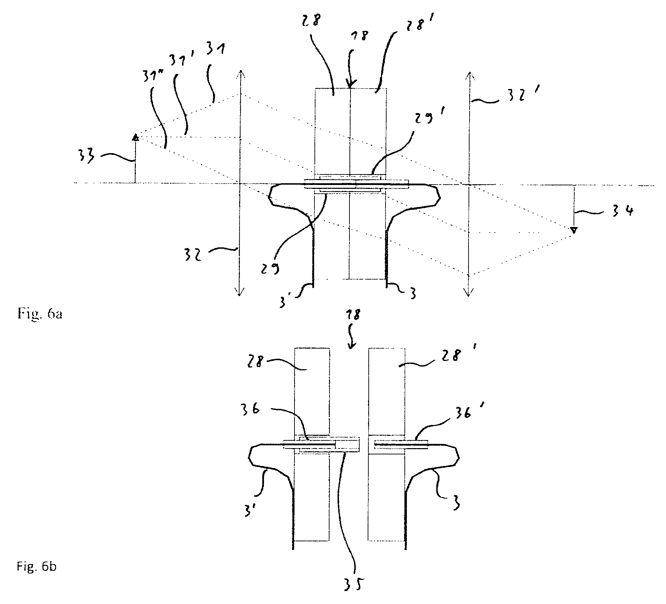

Die Fenster 28 und 28' weisen zentrale Bohrungen 29, 29' auf, deren Achsen im Wesentlichen koaxial zur Drehachse angeordnet ist, und die einen zentralen Kanal zur Übertragung des Beleuchtungslichts bilden. Durch die Bohrungen 29, 29' sind eine Lichtleitfaser 3 vom Kameramodul 11 her und eine Lichtleitfaser 3' von der Endoskopoptik 10 her geführt. Durch entsprechende Fassungen und Führungselemente, für deren Erläuterung auf Fig. 6a und 6b verwiesen wird, wird bei der Verbindung der Endoskopoptik 10 mit dem Kameramodul 11 eine lichtleitende und drehbare Verbindung zwischen den Lichtleitfasern 3, 3' hergestellt. Die Lichtleitfasern 3, 3' können dabei jeweils insbesondere als Multimode-Faser ausgebildet sein, aber auch als Singlemode-Faser oder als Faserbündel.

Im Bereich des parallelen Strahlengangs verursachen die Zuführungen der Lichtleitfasern 3, 3' in den axialen Kanal 19 sowie die optischen und mechanischen Elemente innerhalb der Bohrungen 29, 29' bzw. innerhalb des zentralen Kanals zwar einen geringfügigen Helligkeitsverlust, jedoch ansonsten keine weiteren wesentlichen Störungen des auf dem Bildaufnehmer 2 aufgenommenen Bilds. Um den Helligkeitsverlust möglichst gering zu halten, ist im proximalen Endbereich der Endoskopoptik eine Aufweitungsoptik 30 angeordnet, die einen parallelen Strahlengang mit vergrößertem Durchmesser erzeugt. Die Abbildungsoptik des Kameramoduls 11, d. h. die Kameralinse 13, die gemäß Fig. 5a und 5b auch eine Anordnung aus mehreren abbildenden optischen Elementen sein kann, ist der Aufweitung des Strahlengangs entsprechend angepasst.

Die in Fig. 5a und 5b beispielhaft dargestellte Endoskopoptik 10 ist eine Seitblickoptik, bei der im Bereich des Endoskopobjektivs 8 ein Umlenkelement 26, beispielsweise ein Umlenkspiegel oder ein Umlenkprisma, vorgesehen ist, um eine um 90° abgewinkelte Blickrichtung 17 zu erzielen. Der Fluoreszenzumsetzer 6 ist hier zur Beleuchtung eines seitlich angeordneten Objektfelds 21 angeordnet. Für die weitere Beschreibung des Endoskops wird auf Fig. 1 bis 3 verwiesen. Selbstverständlich kann beispielsweise auch ein Schrägblickendoskop, wie es in Fig. 1 und Fig. 3 gezeigt ist, oder ein Geradeausblickendoskop eine gemäß Fig. 5a und 5b oder den nachfolgenden Figuren ausgebildete Koppelstelle aufweisen.

Wie in Fig. 6a in schematisierter Form gezeigt, wird im Bereich der Koppelstelle 18 das endoskopisch aufgenommene Bild durch die als planparallele Platten ausgebildeten Fenster 28, 28' übertragen. Der Strahlengang ist in diesem Bereich parallel, wie durch die zueinander parallelen Strahlen 31, 31', 31" dargestellt ist. Durch die abbildenden optischen Elemente der Endoskopoptik 10, die in Fig. 6a symbolisch als ideale Sammellinsen 32, 32' dargestellt sind, wird ein Gegenstand 33 in ein Bild 34 abgebildet. Innerhalb der Bohrung 29 ist eine Zentrierhülse 35 angeordnet, die eine Ferrule 36 aufnimmt, die das proximale Ende des Lichtleiters 3' der Endoskopoptik hält. In der Bohrung 29 ist eine Ferrule 36' mit nicht dargestellten Halteelementen gehalten, die das distale Ende des Lichtleiters 3 des Kameramoduls hält. Die Ferrulen 36, 36' und die Zentrierhülse 35 können zur Selbstzentrierung mit Spiel gelagert sein. Die Zentrierhülse 35 kann zum Schutz der optischen Kontaktfläche etwas über die Ferrule 36 vorstehen, während die Gegenferrule 36' etwas gegenüber der Fläche des Fensters 28' zurückversetzt sein kann.

Während bei einer Trennung von Endoskopoptik 10 und Kameramodul 11 die Endflächen der Lichtleiter 3, 3' nicht miteinander in Kontakt sind (Fig. 6b), entsteht bei einer Verbindung der Endoskopoptik 10 mit dem Kameramodul 11 ein faseroptischer Kontakt, der eine weitgehend verlustfreie Übertragung des Beleuchtungslichts vom Kameramodul 11 in die Endoskopoptik 10 ermöglicht (Fig. 6a). Zur Verbesserung des Kontakts können Federelemente vorgesehen sein, die die Ferrulen 36, 36' in entsprechender Richtung vorbelasten (nicht dargestellt).

Zur Vermeidung von Streulicht sind die Ferrulen 36, 36' lichtdicht ausgebildet. Weiterhin können beispielsweise Metallkapillaren zur Führung der Fasern vorgesehen sein und/oder die Fasern sind lichtdicht beschichtet, beispielsweise metallisiert. Die Ferrulen 36, 36' und die Zentrierhülsen 35 werden vorteilhafterweise aus einem lichtundurchlässigen Material hergestellt, beispielsweise aus Metall oder aus lichtundurchlässiger Keramik. Ferner können Montagekomponenten vorgesehen sein, die die Bohrungen und damit die jeweiligen Innerräume von Endoskopoptik 10 und Kameramodul 11 flüssigkeits- und/oder gasdicht abdichten (nicht dargestellt). Um bei einer Drehung eine Reibung von Glasflächen aufeinander zu vermeiden, kann mindestens eine der Kontaktflächen der Ferrulen 36, 36' mit einem leichten Konkavschliff versehen sein. Es kann eine der Ferrulen 36, 36' auch leicht schräg geschliffen sein (nicht dargestellt). Wie Fig. 6a zeigt, entsteht durch die optischen und mechanischen Bauelemente für die faseroptische Verbindung zwar eine Abschattung, diese ist jedoch nur geringfügig, da der durch die Bohrungen 29, 29' gebildete zentrale Kanal nur einen geringen Teil der zur Lichtübertragung genutzten Querschnittsfläche des axialen Kanals einnimmt. Hierbei ist zu berücksichtigen, dass die Strahlen 31' und 31" geringfügig außerhalb der Zeichenebene liegen und daher nicht abgeschattet werden.

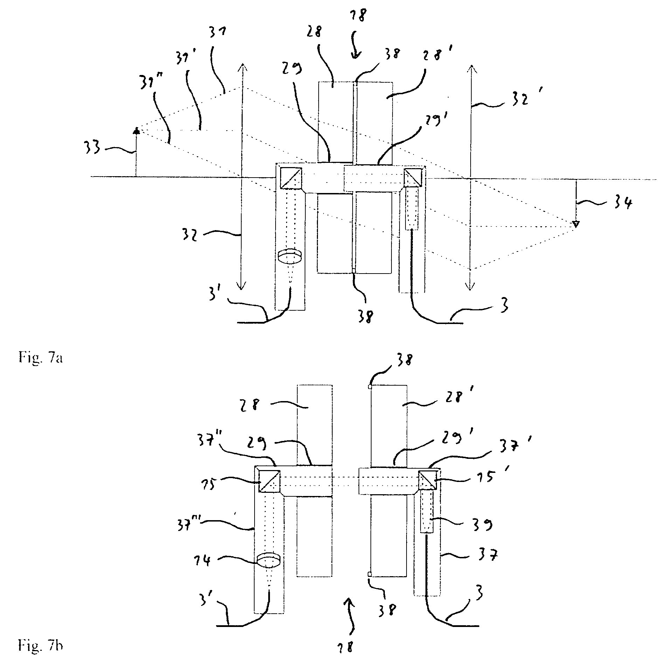

Fig. 7a und 7b zeigen in wiederum schematisierter Darstellung eine Schnittstelle gemäß einem weiteren Ausführungsbeispiel. Dabei zeigt Fig. 7a die Koppelstelle in verbundener Stellung mit angedeutetem Strahlengang der Bildübertragung, der dem der Fig. 6a entspricht, sowie der Beleuchtungslichtübertragung, und Fig. 7b in getrennter Stellung mit lediglich zur detaillierteren Erläuterung angedeutetem Beleuchtungsstrahlengang. In diesem Ausführungsbeispiel sind die Lichtleiter 3, 3' nicht bis an die Koppelstelle 18 geführt, sondern das Beleuchtungslicht wird durch den Lichtleiter 3' zugeführt und, beispielsweise über eine GRIN-Linse (Gradientenindex-Linse) 39 parallel kollimiert und auf einen Umlenkspiegel 15', der als Umlenkprisma ausgebildet sein kann, geleitet. Das kollimierte Licht gelangt nach dem Durchtritt durch die Koppelstelle 18 auf einen weiteren Umlenkspiegel 15, der ebenfalls als Umlenkprisma ausgebildet sein kann. Von dort gelangt das Beleuchtungslicht zu einer Fokussierlinse 14, die in Fig. 7a und 7b als Achromat dargestellt ist, die das Licht in die Lichtleitfaser 3' bündelt. Hierfür können prinzipiell auch Gradientenindex-, sphärische oder asphärische Linsen sowie diffraktive Elemente verwendet werden. Um Lichtstreuung und damit eine Störung des übertragenen Bilds zu verhindern, sind beidseits der Koppelstelle opake Rohre 37, 37', 37", 37'" vorgesehen, die eine lichtdichte Kapselung des Beleuchtungslichtwegs darstellen, und die an der Koppelstelle ineinandergreifen können, und die in den Bohrungen 29, 29' der Fenster 28, 28' befestigt sind. Um ein Verkratzen der planparallelen Platten der Fenster 28, 28' bei einer Drehung des Kameramoduls 11 gegenüber der Endoskopoptik 10 zu verhindern, können hier, wie auch bei den anderen Ausführungsbeispielen, die Fenster 28, 28' durch Abstandshalter 38 leicht getrennt sein. Ebenso ist die Verwendung eines Indexausgleichsmediums vorteilhaft. Die Fenster 28, 28' könnten zur Vermeidung von Beschädigungen bei der Rotation auch gekrümmte Flächen aufweisen oder mit einer Schutz- oder Gleitschicht versehen sein.

### Bezugszeichenliste

1 Diodenlaser
2 Elektronischer Bildaufnehmer
3 3' Lichtleiter
4 4' Strahlteiler
5 Optisches Bildübertragungssystem
6 Fluoreszenzumsetzer
6' Reflektor
7 Versorgungs- und Datenleitungen
8 Endoskopobjektiv
9 Stablinse
10 Endoskopoptik
11 Kameramodul
12 Achse
13 Kameralinse
14 Fokussierlinse
15 15' Umlenkspiegel
16 Schaft
17 Blickrichtung
18 Koppelstelle
19 Axialer Kanal
20 Zapfen
21 Objektfeld
22 Gehäuse
23 Strahlteilende Stablinse
24 Teilerschicht
25 Strahlaustritt
26 Umlenkelement
27 Haltevorrichtung
28 28' Fenster
29 29' Bohrung
30 Aufweitungsoptik
31 31' 31" Strahl
32 32' Sammellinse
33 Gegenstand
34 Bild
35 Zentrierhülse
36 36' Ferrule
37 37' 37" 37'" Rohr
38 Abstandshalter
39 GRIN-Linse

## Patentansprüche

1. Endoskop, umfassend
- eine in einen Hohlraum einführbare Endoskopoptik (10), die ein Lichtübertragungssystem zur Übertragung von Beleuchtungslicht zur Beleuchtung eines im Hohlraum angeordneten Objektfelds (21) und ein optisches Bildübertragungssystem (5) zur Übertragung eines Bilds des Objektfelds (21) aufweist, und
- ein Kameramodul (11) mit einem elektronischen Bildaufnehmer zur Aufnahme des Bilds des Objektfelds (21) und einer Lichtzuführung zur Zuführung von Beleuchtungslicht,
- wobei Endoskopoptik (10) und Kameramodul (11) an einer Koppelstelle (18) lösbar und bezüglich einer Drehachse drehbar miteinander verbunden sind,
wobei im Bereich der Koppelstelle (18) ein axialer Kanal (19) zur Übertragung des Bilds des Objektfelds (21) von der Endoskopoptik (10) zum Kameramodul (11) und zur Übertragung des Beleuchtungslichts vom Kameramodul zur Endoskopoptik (10) vorgesehen ist, wobei besagtes Bild des Objektfelds (21) in einem Bereich des axialen Kanals übertragen wird, der einen zentralen Bereich des axialen Kanals radial umgibt, und wobei besagtes Beleuchtungslicht in einem zentralen Bereich des axialen Kanals vom Kameramodul zur Endoskopoptik übertragen wird,
**gekennzeichnet dadurch**, das
der zentrale Kanal im Bereich der Koppelstelle (18) lichtdicht koppelbar ausgebildet ist und dass das Beleuchtungslicht innerhalb des zentralen Kanals über Lichtleitfasern übertragen wird, deren Endflächen federnd gegeneinander vorgespannt sind.

2. Endoskop nach Anspruch 1,
**dadurch gekennzeichnet, dass** die Koppelstelle (18) im Bereich eines parallelen Strahlengangs des Bildübertragungssystems (5) und/oder einer Eintrittspupille des Kameramoduls (11) angeordnet ist.

3. Endoskop nach einem der Ansprüche 1 bis 2,
**dadurch gekennzeichnet, dass** die Endoskopoptik (10) eine Aufweitungsoptik (30) zur Aufweitung eines Strahlengangs des Bildübertragungssystems (5) aufweist.

4. Endoskop nach einem der vorhergehenden Ansprüche, **dadurch gekennzeichnet, dass** das Beleuchtungslicht in einem ersten Spektralbereich und das Bild des Objektfelds (21) in einem hiervon getrennten zweiten Spektralbereich durch den axialen Kanal (19) übertragbar sind.

5. Endoskop nach Anspruch 4,
**dadurch gekennzeichnet, dass** die Endoskopoptik (10) einen ersten Strahlteiler (4) umfasst zur Auskopplung des Beleuchtungslichts aus dem Bildübertragungssystem (5) und einen Umsetzer zur zumindest teilweisen Umsetzung des Beleuchtungslichts aus dem ersten in den zweiten Spektralbereich, wobei der Umsetzer im Lichtübertragungssystem distal zum ersten Strahlteiler angeordnet ist.

6. Endoskop nach Anspruch 5,
**dadurch gekennzeichnet, dass** der erste Strahlteiler (4) in einem distalen Endbereich oder in einem proximalen Endbereich der Endoskopoptik (10) vorgesehen ist.

7. Endoskop nach einem der Ansprüche 4 bis 6,
**dadurch gekennzeichnet, dass** das Kameramodul (11) einen zweiten Strahlteiler (4') umfasst zur Einkopplung des Beleuchtungslichts in den axialen Kanal (19).

8. Endoskop nach einem der vorhergehenden Ansprüche, **dadurch gekennzeichnet, dass** das Kameramodul (11) eine Halbleiterlichtquelle zur Erzeugung des Beleuchtungslichts aufweist, insbesondere einen Diodenlaser (1) oder eine LED.

9. Endoskop nach einem der vorhergehenden Ansprüche, **dadurch gekennzeichnet, dass** die Lichtzuführung einen Anschluss umfasst zum Anschluss eines Verbindungskabels zur Verbindung mit einer externen Versorgungseinrichtung, wobei das Verbindungskabel mindestens ein elektrisches Kabel zur Versorgung und/oder Datenübertragung des Bildaufnehmers und mindestens ein Lichtleitkabel zur Einspeisung von einer externen Lichtquelle erzeugtem Beleuchtungslicht aufweist.

10. Endoskop nach einem der vorhergehenden Ansprüche, **dadurch gekennzeichnet, dass** mindestens ein Identifikations- bzw. Sicherheitselement vorgesehen ist.

## Claims

1. Endoscope comprising
- an endoscope optical unit (10), which is introducible into a cavity and which has a light transmission system for transmitting illumination light for illuminating an object field (21) arranged in the cavity and an optical image transmission system (5) for transmitting an image of the object field (21), and
- a camera module (11) with an electronic image recorder for recording the image of the object field (21) and a light supply for supplying illumination light,
- wherein the endoscope optical unit (10) and camera module (11) are detachably connected to one another at a coupling point (18) in a manner rotatable with respect to an axis of rotation,
wherein an axial channel (19) for transmitting the image of the object field (21) from the endoscope optical unit (10) to the camera module (11) and for transmitting the illumination light from the camera module to the endoscope optical unit (10) is provided in the region of the coupling point (18), wherein said image of the object field (21) is transmitted in a region of the axial channel which radially surrounds a central region of the axial channel and wherein said illumination light is transmitted from the camera module to the endoscope optical unit in a central region of the axial channel,
**characterized in that**
the central channel has an embodiment that is coupleable in a light-tight manner in the region of the coupling point (18) and **in that** the illumination light is transmitted within the central channel by way of optical fibres, the end faces of which are resiliently pre-tensioned against one another.

2. Endoscope according to Claim 1, **characterized in that** the coupling point (18) is arranged in the region of a parallel beam path of the image transmission system (5) and/or of an entrance pupil of the camera module (11).

3. Endoscope according to either of Claims 1 and 2,
**characterized in that** the endoscope optical unit (10) has an expansion optical unit (30) for expanding a beam path of the image transmission system (5).

4. Endoscope according to one of the preceding claims, **characterized in that** the illumination light is transmittable through the axial channel (19) in a first spectral range and the image of the object field (21) is transmittable through said axial channel in a second spectral range that is separate from said first spectral range.

5. Endoscope according to Claim 4,
**characterized in that** the endoscope optical unit (10) comprises a first beam splitter (4) for decoupling the illumination light from the image transmission system (5) and a converter for at least partly converting the illumination light from the first into the second spectral range, wherein the converter is arranged distally with respect to the first beam splitter in the light transmission system.

6. Endoscope according to Claim 5,
**characterized in that** the first beam splitter (4) is provided in a distal end region or in a proximal end region of the endoscope optical unit (10).

7. Endoscope according to one of Claims 4 to 6,
**characterized in that** the camera module (11) comprises a second beam splitter (4') for coupling the illumination light into the axial channel (19).

8. Endoscope according to one of the preceding claims, **characterized in that** the camera module (11) has a semiconductor light source for generating the illumination light, in particular a diode laser (1) or an LED.

9. Endoscope according to one of the preceding claims, **characterized in that** the light supply comprises a connector for linking a connection cable for connection with an external supply apparatus, wherein the connection cable has at least one electrical cable for supply of and/or data transmission in relation to the image recorder and at least one light-guiding cable for feeding in illumination light generated by an external light source.

10. Endoscope according to one of the preceding claims, **characterized in that** provision is made of at least one identification or security element.

## Revendications

1. Endoscope, comprenant
- une optique d'endoscope (10) pouvant être introduite dans un espace creux, laquelle possède un système de transmission de lumière destiné à transmettre de la lumière d'éclairage servant à éclairer un champ d'objet (21) disposé dans l'espace creux et un système de transmission d'image optique (5) destiné à transmettre une image du champ d'objet (21), et
- un module de caméra (11) doté d'un enregistreur d'image électronique destiné à enregistrer l'image du champ d'objet (21) et d'une arrivée de lumière destinée à l'arrivée de la lumière d'éclairage,
- l'optique d'endoscope (10) et le module de caméra (11) étant reliés entre eux de manière amovible à un point d'accouplement (18) et de manière rotative par rapport à un axe de rotation,
un canal axial (19) destiné à la transmission de l'image du champ d'objet (21) de l'optique d'endoscope (10) au module de caméra (11) et à la transmission de la lumière d'éclairage du module de caméra à l'optique d'endoscope (10) se trouvant dans la zone du point d'accouplement (18), ladite image du champ d'objet (21) étant transmise dans une zone du canal axial qui entoure dans le sens radial une zone centrale du canal axial, et ladite lumière d'éclairage étant transmise dans une zone centrale du canal axial du module de caméra à l'optique d'endoscope,
**caractérisé en ce que**
le canal central est configuré pour pouvoir être accouplé de manière opaque dans la zone du point d'accouplement (18) et **en ce que** la lumière d'éclairage est transmise à l'intérieur du canal central par le biais de fibres optiques dont les surfaces finales sont soumises à une précontrainte élastique l'une contre l'autre.

2. Endoscope selon la revendication 1, **caractérisé en ce que** le point d'accouplement (18) est disposé dans la zone d'un trajet de rayon parallèle du système de transmission d'image (5) et/ou d'une pupille d'entrée du module de caméra (11).

3. Endoscope selon l'une des revendications 1 à 2,
**caractérisé en ce que** l'optique d'endoscope (10) possède une optique d'élargissement (30) destinée à élargir un trajet de rayon du système de transmission d'image (5).

4. Endoscope selon l'une des revendications précédentes, **caractérisé en ce que** la lumière d'éclairage peut être transmise à travers le canal axial (19) dans une première plage spectrale et l'image du champ d'objet (21) dans une deuxième plage spectrale séparée de celle-ci.

5. Endoscope selon la revendication 4, **caractérisé en ce que** l'optique d'endoscope (10) comprend un premier séparateur de faisceaux (4) destiné à découpler la lumière d'éclairage hors du système de transmission d'image (5) et un convertisseur destiné à convertir au moins partiellement la lumière d'éclairage de la première en la deuxième plage spectrale, le convertisseur étant monté dans le système de transmission de lumière de manière distale par rapport au premier séparateur de faisceaux.

6. Endoscope selon la revendication 5, **caractérisé en ce que** le premier séparateur de faisceaux (4) se trouve dans une zone d'extrémité distale ou dans une zone d'extrémité proximale de l'optique d'endoscope (10).

7. Endoscope selon l'une des revendications 4 à 6,
**caractérisé en ce que** le module de caméra (11) comprend un deuxième séparateur de faisceaux (4') destiné à injecter la lumière d'éclairage dans le canal axial (19).

8. Endoscope selon l'une des revendications précédentes, **caractérisé en ce que** le module de caméra (11) possède une source de lumière à semiconducteur servant à générer la lumière d'éclairage, notamment un laser à diode (1) ou une LED.

9. Endoscope selon l'une des revendications précédentes, **caractérisé en ce que** l'arrivée de lumière comprend un raccord destiné à raccorder un câble de liaison servant à la liaison avec un dispositif d'alimentation externe, le câble de liaison possédant au moins un câble électrique destiné à l'alimentation et/ou à la transmission de données de l'enregistreur d'image et au moins un câble à fibre optique destiné à l'injection de la lumière d'éclairage générée par une source de lumière externe.

10. Endoscope selon l'une des revendications précédentes, **caractérisé en ce qu'**il existe au moins un élément d'identification ou de sécurité.
